(19) Europäisches Patentamt
European Patent Office
Office européen des brevets

(11) **EP 2 902 774 B1**

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(51) Int Cl.:
***G01N 27/02*** *(2006.01)*     ***G01N 33/18*** *(2006.01)*
***D06F 39/00*** *(2006.01)*

(21) Anmeldenummer: **14153262.2**

(22) Anmeldetag: **30.01.2014**

(54) **Verfahren und Vorrichtung zur Erfassung von Eigenschaften wässriger Medien durch Impedanzspektroskopie**

Method and device for detecting the characteristics of fluid media by impedance spectroscopy

Procédé et dispositif d'enregistrement des propriétés de fluides aqueux par d'impédance spectroscopie

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**05.08.2015 Patentblatt 2015/32**

(73) Patentinhaber: **Seuffer GmbH & Co. KG**
**75365 Calw (DE)**

(72) Erfinder:
• **Gruden, Roman**
**75180 Pforzheim (DE)**
• **Stephan, Tobias**
**75328 Schömberg (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**US-A1- 2005 179 449**

• **Uwe Tröltzsch ET AL: "Anwendungspotential der Impedanzspektroskopie für die Waschlaugensensorik", , 23. Mai 2012 (2012-05-23), XP055113872, DOI: 10.5162/sensoren2012/ Gefunden im Internet: URL:http://www.ama-science.org/proceedings /getFile/BQN4 [gefunden am 2014-04-14]**
• **HELINANDO P. DE OLIVEIRA ET AL: "Use of Electrical Impedance Spectroscopy as a Practical Method of Investigating the Formation of Aggregates in Aqueous Solutions of Dyes and Surfactants", THE JOURNAL OF PHYSICAL CHEMISTRY B, Bd. 115, Nr. 21, 2. Juni 2011 (2011-06-02) , Seiten 6903-6908, XP055067294, ISSN: 1520-6106, DOI: 10.1021/jp200644y**
• **JORCIN J B ET AL: "CPE analysis by local electrochemical impedance spectroscopy", ELECTROCHIMICA ACTA, ELSEVIER SCIENCE PUBLISHERS, BARKING, GB, Bd. 51, Nr. 8-9, 20. Januar 2006 (2006-01-20), Seiten 1473-1479, XP028027760, ISSN: 0013-4686, DOI: 10.1016/J.ELECTACTA.2005.02.128 [gefunden am 2006-01-20]**

## Beschreibung

Die vorliegende Erfindung betrifft ein Verfahren und eine Vorrichtung zur Erfassung von Eigenschaften wässriger Medien, und insbesondere ein Verfahren und eine Vorrichtung zur Erfassung von Eigenschaften wässriger Medien und darin gelöster Substanzen in Verbindung mit der Impedanzspektroskopie und einer entsprechenden Modellbildung.

In privaten Haushalten und in industriellen Anwendungen besteht im Allgemeinen ein Informationsbedarf hinsichtlich der Eigenschaften fluider Medien. Fluide Medien können hierbei sämtliche fluide Betriebsstoffe sein, wie wässrige Medien in Form wässriger Lösungen von chemischen Substanzen, sowie Öle und dergleichen. Die Erfassung der Eigenschaften der jeweiligen fluiden Medien ist erforderlich zur Bestimmung grundlegender Eigenschaften bei dem Einsatz dieser Medien, die Bestimmung von im Betrieb auftretenden Veränderungen sowie speziell das Auftreten von Verschmutzungen durch Fremdstoffe in einem bestimmten fluiden Medium. Entsprechend den erfassten Messwerten und den daraus durch Auswertung gewonnenen Erkenntnissen kann entschieden werden, ob das fluide Medium im derzeitigen Zustand noch vorbestimmte Anforderungen erfüllt oder ob Maßnahmen zur Reinigung oder zu einem Austausch des fluiden Mediums erforderlich werden.

Ein anschauliches Beispiel eines fluiden Mediums und insbesondere einer wässrigen Lösung ist die in einem Waschgerät (Waschmaschine, Wascheinrichtung) aufbereitete Waschlauge zur Reinigung von Bekleidung oder anderen Gegenständen. Im Hinblick auf Untersuchungen an einer Waschlauge werden nachstehend bekannte Verfahren und Vorrichtungen genannt.

Die Druckschrift DD 217 557 A1 offenbart ein Verfahren und eine zugehörige Vorrichtung zur Regelung der Reinigungs- oder Spülmittelzugabe in Waschgeräten, wobei in dem Waschgerät zur physikalisch-chemischen Bestimmung der Waschlauge unterschiedliche Sensoren angeordnet sind. Über eine der Auswertung der Ausgangssignale der Sensoren dienenden elektronischen Schaltung wird eine Änderung des Anstiegs eines Mess-Signals während der Zugabe von Reinigungs- oder Spülmitteln erfasst und weiter verarbeitet. In Verbindung mit der Auswertung kann hieraus die Waschmitteldosierung für das Waschgerät gesteuert werden.

Die Druckschrift DE 197 55 418 A1 offenbart ein Sensorelement sowie eine Vorrichtung zur Messung komplexer Impedanzen in Materialien, wobei das Sensorelements zwei Elektroden aufweist, die aus einem leitfähigen Material bestehen und die in einem vorbestimmten Abstand zueinander angeordnet sind. Die beiden Elektroden sind mit einer dünnen Isolierschicht überzogen, die eine im Vergleich zu dem vorbestimmten Abstand geringe Schichtdicke aufweist. Das derart gebildete Sensorelement ist gegenüber den Umgebungsbedingungen, deren Eigenschaften erfasst werden sollen, weitgehend unempfindlich. In einer Auswerteschaltung werden die Ausgangssignale der Sensoren einer weiteren Verarbeitung unterzogen, und es kann bei dieser Verarbeitung eine Analyse der Eigenschaften einer jeweils zwischen den Elektroden angeordneten Flüssigkeit durchgeführt werden. Hierbei können insbesondere komplexe Impedanzen als Maß für die Eigenschaften der Flüssigkeiten bestimmt und ausgewertet werden.

Die Druckschrift DE 41 11 064 A1 offenbart ein Verfahren zur Messung der Schmutzbefrachtung einer Waschflotte, wobei Sensoren vorgesehen sind zur Erfassung von Eigenschaften der Waschflotte in Verbindung mit einer Leitfähigkeitsmessung oder einer Messung mittels Impedanzspektroskopie. Ermittelt werden hierbei die Eigenschaften der Waschflotte im Hinblick darauf, eine Differenz zwischen Messwerten bei unbefrachteter Waschflotte und bei verschmutzter Waschflotte zu erhalten und entsprechende Maßnahmen zu ergreifen, wenn eine Erneuerung der Waschflotte aufgrund der Messwerte erforderlich ist. Hierbei werden entsprechende Grenzwerte berücksichtigt.

Schließlich offenbart die Druckschrift DE 603 07 108 T2 ein Verfahren zur Analyse eines Arbeitsfluids unter Verwendung der Impedanzspektroskopie, wobei ein zu erfassendes Fluid mittels Impedanzspektroskopie über einen Bereich von Frequenzen und über eine Mehrzahl von Einzelfrequenzen gemessen wird und entsprechende Impedanzen ausgewertet werden. Aus zugehörigen Kennlinien der Messewerte und vorbestimmten Kriterien können die Eigenschaften des Fluids bestimmt werden.

Die Druckschrift US 2005/0179449 A1 offenbart ein Verfahren zur Analyse eines Schmiermittels unter Verwendung der Impedanzspektroskopie.

U. Tröltzsch et. al. "Anwendungspotential der Impedanzspekroskopie für die Waschlaugensensorik"; 16. GMA/ITG-Fachtagung Sensoren und Messsysteme 2012, S. 650-661 offenbart die Analyse von Waschlaugen mittels Impedanzspektroskopie.

Im Allgemeinen erfolgt beispielsweise die Reinigung von Bekleidung im privaten und industriellen Bereich weitgehend vollautomatisch mit elektronisch gesteuerten Waschgeräten, die auch als Waschvollautomaten bezeichnet werden. Es ist insgesamt ein Ziel eines Waschablaufs, ein optimales Reinigungsergebnis bei minimalem Einsatz von Wasser, elektrischer und thermischer Energie sowie von Waschmitteln zu erzielen. Moderne Waschgeräte weisen beispielsweise optische Messverfahren zur Erfassung der Trübung einer Waschlauge zur zumindest ungefähren Bestimmung einer Waschmittelkonzentration sowie weitere Verfahren auf, wobei jedoch im Allgemeinen eine aufwendige Datenauswertung erforderlich ist. Trübungssensoren weisen oftmals eine geringe Genauigkeit, wobei das Messergebnis leicht durch Ablagerungen im Bereich des Trübungssensors verfälscht werden kann, die in Waschgeräten in Form von Waschmittel- und Schmutzresten häufig auftreten.

Wichtige Größen in Verbindung mit Waschvorgängen sind dabei als grundlegende Größe die Wasserhärte, sowie die Waschmittelkonzentration und die Waschaktivität. Die Wasserhärte bzw. die Wassereigenschaften des in ein betreffendes Waschgerät eingebrachten Wassers (im Allgemeinen so genanntes Frischwasser oder Trinkwasser) wird meist von den kommunalen Wasserwerken als relativ genaue Größe bekanntgegeben, wobei aktuelle Eigenschaften des Wassers aufwändig in einem Labor ermittelt werden. Die Eigenschaften des Wassers können dabei nach Jahreszeit und Wetterbedingungen größeren Veränderungen unterliegen. In Abhängigkeit von diesen Angaben kann eine Waschmittelmenge nach Abschätzung der Verschmutzung des Waschguts bestimmt werden. Es ist dabei erkennbar, dass hierbei lediglich grobe Abschätzungen vorgenommen werden, mit denen eine genaue Dimensionierung einer optimalen Waschmittelmenge kaum möglich ist.

Eine genaue Bestimmung der so genannten Wasserhärte stellt eine wichtige Grundlage für ein wirtschaftliches und effektives Reinigen von Textilien dar, wobei der Begriff der Wasserhärte sich im Wesentlichen auf die Konzentration der im Wasser gelösten Ionen von Erdalkalimetallen bezieht. Die so genannte Gesamthärte betrifft hauptsächlich die Kalzium- und Magnesiumionen als gelöste Stoffe im Wasser, sowie auch deren anionische Partner, wie beispielsweise Hydrogencarbonationen $HCO_3^-$. In einer verallgemeinerten Betrachtungsweise betrifft die Erfassung der Eigenschaften eines fluiden Mediums wie Wasser sämtliche im dem Wasser gelöste Stoffe, sowie auch Stoffe, die den Waschvorgang beeinflussen können.

Die Wasserparameter oder Wassereigenschaften und in diesem Zusammenhang die gelösten Inhaltstoffe (insbesondere die temperaturabhängige Carbonathärte) sind nachteilig für Waschvorgänge und für eine Vielzahl weiterer Prozesse, bei denen das Wasser verarbeitet wird.

Bei kommunalen Wasserwerken wird die Wasserhärte, insbesondere die so genannte Gesamthärte, unter Laborbedingungen durch eine quantitative Analyse in Form einer Titration bestimmt. In einer Probelösung mit einem bekannten Stoff in unbekannter Konzentration wird gezielt ein bekannter Stoff mit bekannter Konzentration (so genannte Maßlösung) hinzugefügt, und es wird das Volumen der verbrauchten Maßlösung bestimmt. Auf der Basis des verbrauchten Volumens der Maßlösung kann auf die unbekannte Konzentration eines bestimmten Stoffes in der Probelösung geschlossen werden.

Es ist erkennbar, dass die quantitative Analyse mittels Titration Laborbedingungen erfordert, die hinsichtlich der nötigen Apparate und Einrichtungen aufwendig sind, und es ist des Weiteren erforderlich, eine in ihrer Konzentration genau bestimmte Maßlösung der Probelösung hinzuzufügen. Die Anwendung der quantitativen Analyse mittels Titration bleibt somit einem gut ausgerüsteten Labor vorbehalten und ist in dieser Form nicht anwendbar für eine einfache und schnelle Erfassung von Eigenschaften eines fluiden Mediums, wie beispielsweise von Wasser, in allgemeinen industriellen Anwendungen oder in privaten Haushalten.

Da zunehmend die Waschvorgänge und die verwendeten Waschmittel unter Umweltschutzgesichtspunkten betrachtet werden, ist es erforderlich, sowohl eine Wassermenge als auch eine Waschmittelmenge in Abhängigkeit von dem Verschmutzungsgrad eines Waschguts zu dosieren, wobei ebenfalls die Eigenschaften des Wassers (im Allgemeinen Trinkwasser) zu berücksichtigen sind. Eine gezielte und richtige Waschmitteldosierung führt einerseits zu einer Einsparung an Energie und Wasser, sowie andererseits auch zu einer geringeren Belastung des Abwassers. Zu dem Kriterium der Verschmutzung des Waschguts kommt nun das Kriterium der Eigenschaften des Wassers hinzu, da diese erheblichen Einfluss haben und bei der Waschmitteldosierung berücksichtigt werden müssen.

Bei der Dosierung von Waschmitteln für Waschvorgänge wird im allgemeinen ein gewisser Prozentsatz von Waschmitteln zuviel dosiert, so dass einerseits ein erhöhter Energiebedarf für die Herstellung dieser Waschmittelmenge und andererseits eine Umweltbelastung vorliegt, die durch eine verbesserte Dosierung vermieden werden kann. Eine erhöhte Dosierung des Waschmittels führt zu einer größeren benötigten Wassermenge durch häufigeres und/oder längeres Spülen, wobei die gesamten Waschzeiten ansteigen und mehr Energie benötigt wird. Automatische Dosiereinrichtungen können hierbei eine Lösung darstellen, insbesondere wenn die Eigenschaften eines zugeführten Frischwassers bestimmt werden können. Eine besondere Rolle bei der Waschmitteldosierung kommt der Wasserhärte zu. Ein Wasser mit großer Wasserhärte, d.h. mit vielen gelösten Stoffen, führt zur Bildung sogenannte Kalkseife, die keine Reinigungswirkung entfaltet. Vielmehr kann sich die Kalkseife auf Oberflächen wie beispielsweise auf dem Waschgut oder in Abwasserrohren absetzen und Ablagerungen bilden. Somit kann eine verbesserte Dosierung im Wesentlichen nur dann erreicht werden, wenn zumindest ungefähr die Wasserhärte bekannt ist.

Bisher eingesetzte Trübungssensoren liefern keine Rückschlüsse über die Wasserhärte, beispielsweise über einen Gehalt an Kalzium- und Magnesiumionen im Wasser, da diese härtebildenden Ionen keine Trübung des Wassers verursachen. Weitere Möglichkeiten bestehen in der Erfassung der elektrischen Eigenschaften des Wassers, beispielsweise über eine Leitwertmessung. Insgesamt ist jedoch eine einerseits zuverlässige und andererseits kostengünstige Erfassung des Kalzium- und Magnesiumgehalts im Wasser erforderlich. Hierbei kann das grundsätzliche Messprinzip in Verbindung mit der Impedanzspektroskopie Vorteile bringen. Die Messung eines Impedanzverlaufs eines kapazitiven Elements, dessen elektrische und speziell kapazitive Eigenschaften durch das Prozessmedium, wie beispielsweise ein Waschwasser, veränderlich sind, kann in der Auswertung Angaben über Gesamtwasserhärte, Waschmittelgehalt und Verschmutzungsgrad sowie weitere spezielle Eigenschaften ermöglichen. Es kann ferner die Gesamthärte des Wassers in eine sog. Carbonathärte und eine sog. Nicht-Carbonathärte unterteilt werden. Da Karbonate (im wesentlichen Kalziumkarbonate) an verschiedenen Elementen einer Wascheinrichtung Ablagerungen bilden können, ist eine Unterteilung in Karbonat- und Nicht-Carbonathärte zur detaillierten Analyse des fluiden Mediums, wie beispielsweise eines Frischwassers, sinnvoll.

Die gesamte Sensoreinrichtung sollte mit großen Vorteilen und auf einfache Weise in jede Wascheinrichtung eingesetzt werden können, so dass in diesen Wascheinrichtungen eine genaue Bestimmung der Eigenschaften des Frischwassers und somit auch eine genaue Dosierung einer Waschmittelmenge ohne Eingriffe eines Benutzers möglich ist. Es besteht des weiteren das Erfordernis eines einfachen und kostengünstigen Aufbaus einer derartigen Einrichtung, vorzugsweise in Form eines Moduls, in Verbindung mit einer unkomplizierten und damit ebenfalls kostengünstigen Montage, da bei der weitgehenden Anwendung in Wascheinrichtungen mit größeren Stückzahlen zu rechnen ist.

Der vorliegenden Erfindung liegt daher die Aufgabe zugrunde, ein Verfahren und eine Vorrichtung zur Erfassung von Eigenschaften wässriger Medien derart auszugestalten, dass auf einfache Weise und mit einem verminderten Aufwand eine genaue und kontinuierliche Messung der Eigenschaften der wässrigen Medien in Verbindung mit einer überschaubaren Auswertung gewährleistet ist.

Erfindungsgemäß wird diese Aufgabe bezüglich eines Verfahrens zur Erfassung von Eigenschaften wässriger Medien sowie einer Vorrichtung zur Durchführung des Verfahrens mit den in den beigefügten Patentansprüchen angegebenen Merkmalen gelöst.

Das erfindungsgemäße Verfahren zur Erfassung von Eigenschaften wässriger Medien mittels einer Sensoreinrichtung und in Verbindung mit einer Impedanzspektroskopie, umfasst die Schritte: Bereitstellen eines Modells der Sensoreinrichtung und des zu erfassenden Mediums mit jeweiligen Modellparametern (Schritt S1), Ermitteln eines Impedanzspektrums einer vorbestimmten Probe des Mediums über einen vorbestimmten Frequenzbereich (Schritt S2), Bestimmen der Modellparameter auf der Basis der Daten des Impedanzspektrums und Anwenden der Parameter als Werte in dem Modell (Schritt S3), Bilden von Differenzwerten zwischen Daten aus dem Modell mit den Modellparametern und erfassten Daten des Impedanzspektrums einer weiteren Probe des Mediums über den vorbestimmten Frequenzbereich (Schritt S4), und Ermitteln eines Maximalwerts der Differenzwerte und Bestimmen der Frequenz des Maximalwerts aus dem vorbestimmten Frequenzbereich als Maß für die spezifische Eigenschaft des Mediums (Schritt S5).

Die erfindungsgemäße Vorrichtung umfasst sämtliche zur Durchführung des Verfahrens erforderlichen Einrichtungen.

Mit der erfindungsgemäßen Lösung ist es möglich, unterschiedliche Eigenschaften fluider Medien, und insbesondere wässriger Medien zu bestimmen, wie beispielsweise in Verbindung mit einer wässrigen Lösung oder von Frischwasser, wobei die Konzentration einer Carbonathärte (Hydrogencarbonationen-Konzentration $c(HCO3^-)$) bestimmt wird. Auf der Basis der bekannten Konzentration können weitere Maßnahmen bestimmt werden, wie die Menge einer Waschmittelkonzentration oder die Möglichkeiten einer Beseitigung oder Verminderung der Carbonathärte mittels anderer Einrichtungen, Verfahren und Substanzen.

Das Verfahren und die Vorrichtung zur Erfassung der Eigenschaften wässriger Medien gemäß der vorliegenden Erfindung ermöglicht somit auf einfache Weise eine genaue und kontinuierliche Erfassung der Eigenschaften des Mediums und speziell der Carbonathärte, wobei die zugehörige Sensoreinrichtung dem Medium ausgesetzt ist bzw. von dem Medium umspült ist, und die Erfassung mittels einer entsprechenden Ansteuerung der Sensoreinrichtung mittels der Impedanzspektroskopie ohne Eingriffe eines Benutzers erfolgt. Hierbei können entsprechende Kennlinien ermittelt werden, aus denen charakteristische Daten entsprechend einem zugehörigen physikalischen Modell bestimmt werden können.

In Kenntnis der Carbonathärte ist es daher möglich, dem Anwender der Vorrichtung wie beispielsweise dem Benutzer eines Haushaltsgeräts oder auch einer Einrichtung in einer industriellen Anwendung oder einer kommunalen Anlage schnell eine gewünschte Information zur verschaffen, aus der die erfasste Carbonathärte hervorgeht, so dass gezielt weitere Steuerungs- oder Regelungsmaßnahmen ergriffen werden können, mittels denen ein bestimmter Prozess, beispielsweise ein Wasch- oder Reinigungsvorgang, optimiert werden kann. Dies betrifft beispielsweise die Dosierung von Waschmittel im Wasser einer Wascheinrichtung, wenn das Wasser eine bestimmte Carbonathärte aufweist. In Verbindung mit einer einfachen Sensoranordnung eröffnet dies die Möglichkeit, das Verfahren und die Vorrichtung gemäß der vorliegenden Erfindung in einem automatisierten System bei einer industriellen Anwendung oder auch in Haushaltsgeräten einzusetzen, wobei jeweilige Benutzer keine zusätzlichen Maßnahmen ergreifen müssen oder eine weitere Schulung oder Einweisung benötigen. Entsprechende Prozesse können teilweise automatisiert oder vollautomatisch ablaufen.

Als Grundlage wird ein Modell oder elektrochemisches Ersatzschaltbild bestimmt, wobei das Modell das elektrische Verhalten des Sensors sowie die chemischen Eigenschaften der zu untersuchenden fluiden Mediums (beispielsweise der wässrigen Lösung) bezeichnen.

Beide Ausgestaltungen der vorliegenden Erfindung sind in den Unteransprüchen angegeben.

In dem Verfahren kann das Modell ein Constant-Phase-Element aufweisen zur modellmäßigen Darstellung der Eigenschaften der Sensoreinrichtung (Sensorzelle).

Die Modellparameter des Modells können einen ersten Parameter zur Berücksichtigung einer Doppelschicht-Kapazität der Sensoreinrichtung und einen zweiten Parameter zur Berücksichtigung eines Phasenwinkels des Impedanzspektrums in dem vorbestimmten Frequenzbereich aufweisen.

Das Ermitteln des Impedanzspektrums kann das Ermitteln von Impedanzwerten der Sensoreinrichtung unter dem Einfluss des Mediums im vorbestimmten Frequenzbereich umfassen.

Der Schritt (S4) des Bildens von Differenzwerten kann den Schritt des Simulierens des Modells mit den bestimmten Modellparametern in dem vorbestimmten Frequenzbereich, und das Erfassen von Impedanzwerten eines Impedanzspektrums einer Probe des Mediums in dem vorbestimmten Frequenzbereich umfassen.

Der Schritt (S4) des Bildens von Differenzwerten kann den Schritt des Bildens der Differenzwerte aus den simulierten Daten des Modells und den erfassten Impedanzwerten der Proben des Mediums für eine jeweilige Frequenz aus dem vorbestimmten Frequenzbereich umfassen.

Das Modell kann eine Parallelschaltung eines Widerstandselements zur Berücksichtigung des Elektrolytwiderstands sowie eines kapazitiven Elements zur Berücksichtigung der Elektrolytkapazität des Mediums aufweisen.

In dem Verfahren kann zur Überprüfung mehrere Proben des Mediums jeweils eine Wiederholung der Schritte S1 bis S5 erfolgen.

Die Erfindung wird nachstehend anhand von Ausführungsbeispielen unter Bezugnahme auf die Zeichnung näher beschrieben.

Es zeigen:

Fig. 1 ein Blockschaltbild einer Schaltungsanordnung (Vorrichtung) zur Durchführung einer Impedanzspektroskopie,

Fig. 2 eine vereinfachte schematische Darstellung der Vorrichtung gemäß Fig. 1, die beispielsweise in einem Gerät verwendet und dort angeordnet ist,

Fig. 3 eine mittels Impedanzspektroskopie erhaltene Kennlinie K der Impedanz Z mit der Darstellung des Imaginärteils Im(Z) über dem Realteil Re(Z),

Fig. 4 eine schematische Darstellung einer Sensorzelle in Form eines Plattenkondensators mit dem physikalischen Modell (elektrochemisches Ersatzschaltbild),

Fig. 5 eine grafische Darstellung einer Kennlinie zum Vergleich von erfassten Daten einer bekannten Wasserprobe mit Daten des Modells,

Fig. 6 eine grafische Darstellung einer Kennlinie zur Veranschaulichung von Differenzen zwischen Daten einer bekannten Wasserprobe und den Daten des physikalischen Modells, und

Fig. 7 ein Ablaufdiagramm zur Veranschaulichung des Verfahrens mit einzelnen Verfahrensschritten.

Beschreibung bevorzugter Ausführungsbeispiele:

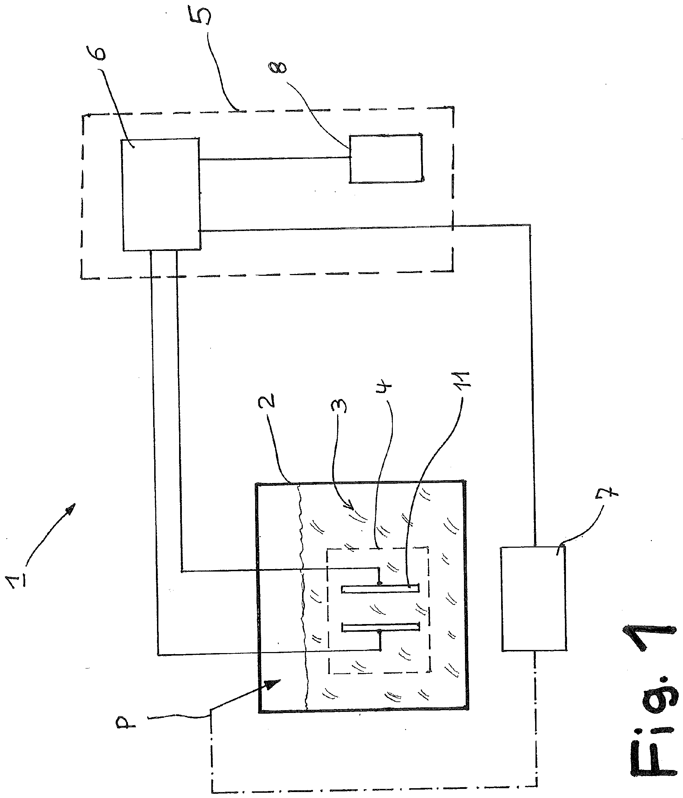

Nachstehend werden anhand des in Fig. 1 gezeigten schematischen Blockschaltbilds der Aufbau und die Wirkungsweise des erfindungsgemäßen Verfahrens sowie der erfindungsgemäßen Vorrichtung gemäß einem Ausführungsbeispiel beschrieben.

Fig. 1 zeigt die Vorrichtung 1 zur Erfassung der Eigenschaften fluider Medien (nachstehend vereinfacht als "die Vorrichtung 1" bezeichnet), wobei die Vorrichtung 1 in einem Behälter 2 mit Kontakt zu einem dort befindlichen fluiden Medium 3 angeordnet ist. Der Begriff des Behälters ist hierbei in sehr allgemeiner Form aufzufassen, wobei es sich beispielsweise um einen Tank in einer Maschine, eine Trommel einer Wascheinrichtung (Waschmaschine) oder einen beliebigen Behälter mit Frischwasser oder einer andere wässrigen Lösung handeln kann. Die vorliegende Erfindung ist nicht auf eine dieser Behälterarten beschränkt.

In jedem Fall beinhaltet der Behälter 2 das zu erfassende fluide Medium 3, beispielsweise ein Frischwasser aus einer kommunalen Wasserversorgungseinrichtung (Wasserwerk), in einer ausreichenden Menge, so dass es mittels einer Sensoreinrichtung 4 hinsichtlich seiner Eigenschaften erfasst werden kann. Die Sensoreinrichtung 4 ist daher ganz oder zumindest teilweise von dem Medium 3 umgeben bzw. umströmt, wobei die Sensoreinrichtung 4 so weit von dem Medium umgeben sein muss, dass eine ausreichend verlässliche und nachvollziehbare Messung möglich ist. Hierbei ist der Begriff des Mediums ebenfalls sehr allgemein im Sinne einer wässrigen Lösung auszulegen. Zur Vereinfachung der Darstellung wird nachstehend ohne eine Beschränkung hierauf die vorliegende Erfindung in Verbindung mit der Überprüfung der Eigenschaften von Wasser oder wässrigen Lösungen beschrieben, d. h. anhand von Lösungen, die zur Ionenbildung fähig sind.

Die Vorrichtung 1 gemäß Fig. 1 umfasst ferner eine Steuerungseinrichtung 5, die einerseits zur Steuerung und/oder Regelung der gesamten Abläufe der Erfassung mittels der Impedanzspektroskopie dient, und andererseits eine Ansteuerung der Sensoreinrichtung 4 veranlasst, sowie eine Auswertung der von der Sensoreinrichtung 4 abgegebenen Signale (Erfassungssignale) durchführt.

Zu diesem Zweck umfasst die Steuerungseinrichtung 5 eine Ansteuerungseinrichtung 6, die mit der Sensoreinrichtung 4 verbunden ist und die, entsprechend innerhalb der Steuerungseinrichtung 5 erzeugten Anweisungen und Befehlen, die Sensoreinrichtung 4 mit jeweiligen elektrischen Signalen ansteuert. Beispielsweise werden an die Sensoreinrichtung 4 zur Durchführung entsprechender Messungen Ströme und Spannungen in Verbindung mit vorbestimmten Frequenzen oder Frequenzbereichen angelegt. Die Steuerungseinrichtung 5 kann dabei auch vorgesehen sein zur Erfassung der Temperatur des Mediums 3, und kann hierzu mit zumindest einer - in den Figuren nicht gezeigten - Temperaturerfassungseinrichtung verbunden sein. Alternativ kann die Ansteuerungseinrichtung 6 auch außerhalb der Steuerungseinrichtung 5 als separate Einheit angeordnet sein, wobei die Sensoreinrichtung 4 und die Ansteuerungseinrichtung 6 bei dieser alternativen Anordnung hinsichtlich ihrer Funktion zu einer Messeinrichtung zusammengefasst werden können.

Die Steuerungseinrichtung 5 kann ferner mit einer Steuereinheit 7 verbunden werden, die direkt in Zusammenhang mit Behälter 2 steht. Wird beispielsweise angenommen, dass der Behälter 2 die Trommel einer Wascheinrichtung (Waschgerät, Waschmaschine) ist oder in Verbindung steht mit einem Frischwasserzulauf der Wascheinrichtung, dann stellt die Steuereinheit die eigentliche Einrichtung zur elektronischen Steuerung der Wascheinrichtung dar. In der Wascheinrichtung werden in der entsprechenden programmierten Weise Wassermenge und Waschmittelzulauf sowie Waschtemperaturen und Zeitverlauf eines Waschvorgangs sowie auch Schleuderzeiten und Schleuderdrehzahlen gesteuert. Die Steuerungseinrichtung 5 kann in Abhängigkeit von Messergebnissen in Verbindung mit der Sensoreinrichtung 4 die Steuereinheit 7 dahingehend beeinflussen, dass, abweichend von einem bestimmten Programm, eine größere oder kleinere Wasser- oder Waschmittelmenge eingesetzt oder beispielsweise auch eine Veränderung der Waschtemperatur vorgenommen werden kann. Somit kann durch die Steuerungseinrichtung 5 in Verbindung mit der Steuereinheit 7 eine variable Steuerung oder auch eine Regelung von Einzelfunktionen der Wascheinrichtung vorgenommen werden. Mittels eines Pfeils P ist gemäß Fig. 1 die Einflussnahme der Steuereinheit 7 auf den Waschvorgang schematisch angedeutet.

Die Steuerungseinrichtung 5 umfasst ferner eine Speichereinrichtung 8, in der zur Durchführung der Erfassung der Materialeigenschaften in Verbindung mit der durchzuführenden Impedanzspektroskopie entsprechende Daten und Programme gespeichert sind, auf die innerhalb der Steuerungseinrichtung 5 zugegriffen werden kann.

Hinsichtlich der Wirkungsweise der in Fig. 1 dargestellten Vorrichtung werden der Sensoreinrichtung 4 entsprechend Anweisungen, die innerhalb der Steuerungseinrichtung 5 in Verbindung mit der Ansteuerungseinrichtung 6 erzeugt werden, vorbestimmte Signale der physikalischen Größen, wie Strom und Spannung sowie mit bestimmten Frequenzen oder Frequenzbereichen, zugeführt, und es werden innerhalb der Steuerungseinrichtung 5 über die Ansteuerungseinrichtung 6 die Erfassungssignale der Sensoreinrichtung 4 aufgenommen und verarbeitet, gegebenenfalls in Verbindung mit aus der Speichereinrichtung 8 ausgelesenen Programmen oder Daten. In der Speichereinrichtung 8 können Grunddaten oder bei früheren Messungen erzeugte Erfassungsdaten gespeichert werden, so dass beispielsweise ein Vergleich aktuell gemessener Daten mit Grunddaten hinsichtlich der Eigenschaften des erfassten fluiden Mediums 3 durchgeführt werden kann. In Abhängigkeit von dem Erfassungsergebnis einerseits und einem Vergleichsergebnis andererseits kann im Falle einer Wascheinrichtung die Steuereinheit 7 angewiesen werden, bestimmte Betriebsparameter (Waschmittelkonzentration, Wassermenge, Temperatur) zu ändern oder beizubehalten.

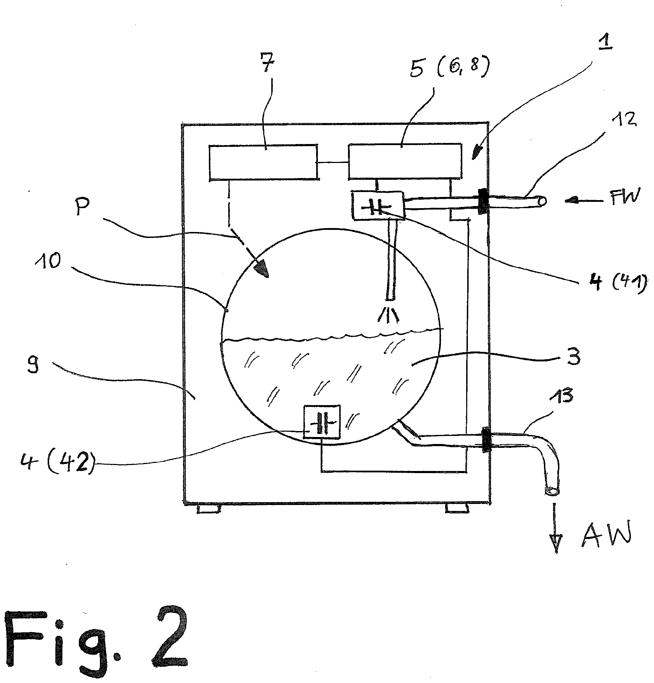

Gemäß Fig. 1 ist die Vorrichtung 1 zur Erfassung der Eigenschaften fluider Medien 3 einschließlich der Sensoreinrichtung 4 und der Steuerungseinrichtung 5 in allgemeiner Form dargestellt. Fig. 2 zeigt demgegenüber die Anordnung der Vorrichtung in der Anwendung in einer Wascheinrichtung 9.

In einer Trommel 10 bzw. einem Laugenbehälter der Wascheinrichtung 9 befindet sich das fluide Medium 3, und vorzugsweise in einem tieferen Bereich der Trommel 10 ist die Sensoreinrichtung 4 angeordnet und steht mit der Steuerungseinrichtung 5 in Verbindung, die die Ansteuerungseinrichtung 6 sowie die Speichereinrichtung 8 umfasst. Die Steuerungseinrichtung 5 ist mit der Steuereinheit 7 der Wascheinrichtung 9 verbunden, und mittels eines Pfeils P ist in Fig. 2 in gleicher Weise wie in Fig. 1 veranschaulicht, dass die Steuereinheit 7 der Wascheinrichtung 9 entsprechende Einflüsse auf den Waschvorgang ausübt.

Gemäß Fig. 2 ist im unteren Teil der Trommel 10 oder auch eines in der Figur nicht gezeigten Laugenbehälters, in dem die Trommel 10 drehbar gelagert ist, die Sensoreinrichtung 4 angeordnet. Eine gleichartige oder ähnliche Sensoreinrichtung 4 ist ebenfalls vorzugsweise oberhalb der Trommel 10 in Verbindung mit einem Zulaufrohr 12 für Frischwasser FW angeordnet. Die Sensoreinrichtung 4 umfasst somit gemäß der Darstellung in Fig. 2 einen ersten Sensor 41, der im Zulaufrohr für Frischwasser angeordnet ist und in Verbindung mit dieser Position in der Lage ist, die Eigenschaften des Frischwassers FW und insbesondere einen Mineralstoffgehalt, wie beispielsweise die Carbonathärte des Frischwassers FW zu bestimmen. Die Sensoreinrichtung 4 umfasst gemäß Fig. 2 einen zweiten Sensor 42, der im unteren Teil der Trommel 10 angeordnet ist und entsprechend dieser Position in der Lage ist, Eigenschaften einer Waschlauge, wie sie in dem Laugenbehälter gebildet wird, zu erfassen, so dass entsprechend dem Erfassungsergebnis weitere Parameter, wie eine Waschmittelzugabe oder Wasserzugabe bestimmt werden können. Es ist auch möglich, an dieser Stelle in dem Laugenbehälter durch den zweiten Sensor 42 die Eigenschaften von Frischwasser zu erfassen, sofern sich in dem in die Wascheinrichtung neu eingeleiteten Frischwasser noch keine Verschmutzung durch ein Waschgut oder ein Waschmittel befindet.

Die vorliegende Erfindung wird in Verbindung mit der Sensoreinrichtung 4 beschrieben, wobei ohne eine grundsätzliche Beschränkung hierauf die nachfolgende Beschreibung den ersten Sensor 41 und/oder den zweiten Sensor 42 betrifft, mittels denen die Eigenschaften des zugeführten Frischwassers FW vor Beginn des Waschvorgangs bestimmt werden können. Erfolgt die Anwendung der vorliegenden Erfindung auf der Basis lediglich des zweiten Sensors 42 der Sensoreinrichtung 4 in einer Anordnung gemäß Fig. 2 (d. h. an einer Position im unteren Bereich des Laugenbehälters 2), dann ist es erforderlich, mittels eines entsprechenden Algorithmus einen Fremdstoffanteil herauszurechnen, da immer mit Fremdstoffen in dem in die Wascheinrichtung 9 einlaufenden Wasser gerechnet werden muss, beispielsweise durch Waschmittelreste in der Wascheinrichtung oder durch Verschmutzungen des Waschguts, wenn das einlaufende Frischwasser das Waschgut benetzt. Der erste Sensor 41 kann gleich wie der zweite Sensor 42 ausgeführt sein, oder kann hinsichtlich seiner geometrischen Ausführung und der entsprechenden Signalverarbeitung ausgelegt sein zur gezielten Erfassung der Eigenschaften des Frischwassers und speziell der Carbonathärte, wobei dies im Einzelnen nachstehend beschrieben wird. Die vorliegende Erfindung ist somit anwendbar mit einem oder zwei Sensoren der Sensoreinrichtung 4. Zumindest einer der Sensoren 41 oder 42 der Sensoreinrichtung 4 ist vorgesehen.

Wie es in Fig. 2 dargestellt ist, kann die Vorrichtung 1 ohne weiteres in eine bestehende und handelsübliche Wascheinrichtung eingebaut werden, wobei lediglich die Sensoreinrichtungen 4 und die Steuerungseinrichtung 5 berücksichtigt werden müssen. Gemäß Fig. 2 entspricht die Trommel 10 (der dargestellten Wascheinrichtung 9) dem Behälter 2 gemäß Fig. 1, und der zweite Sensor 41 gemäß Fig. 2 entspricht der Sensoreinrichtung 4 gemäß Fig. 1. Das fluide Medium 3 gemäß Fig. 1 wird durch das Frischwasser gebildet, dessen Wasserhärte zu bestimmen ist.

Die erfindungsgemäße Vorrichtung 1 und das zugehörige Verfahren können in gleicher Weise bei einer Geschirrspülmaschine oder dergleichen angewendet werden, bei denen ein Frischwasserzulauf erforderlich ist und die Eigenschaften des Frischwassers zur gezielten Steuerung oder Regelung nachfolgender Prozesse zu bestimmen sind. Ferner können bei jeglichen Anwendungen im kommunalen Bereich oder im industriellen Bereich die Vorrichtung oder das Verfahren verwendet werden, um auf einfache und trotzdem genaue Weise die Wasserhärte zu bestimmen, ohne dass aufwendige Laboreinrichtungen und entsprechende Maßnahmen erforderlich sind.

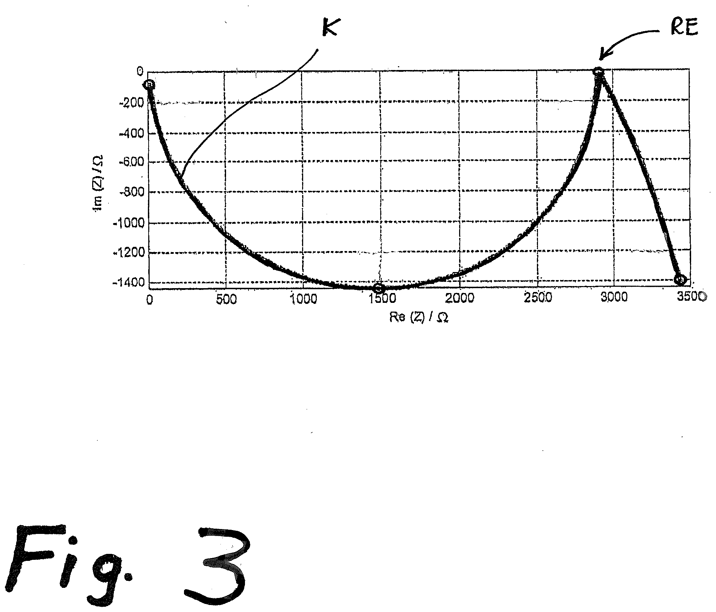

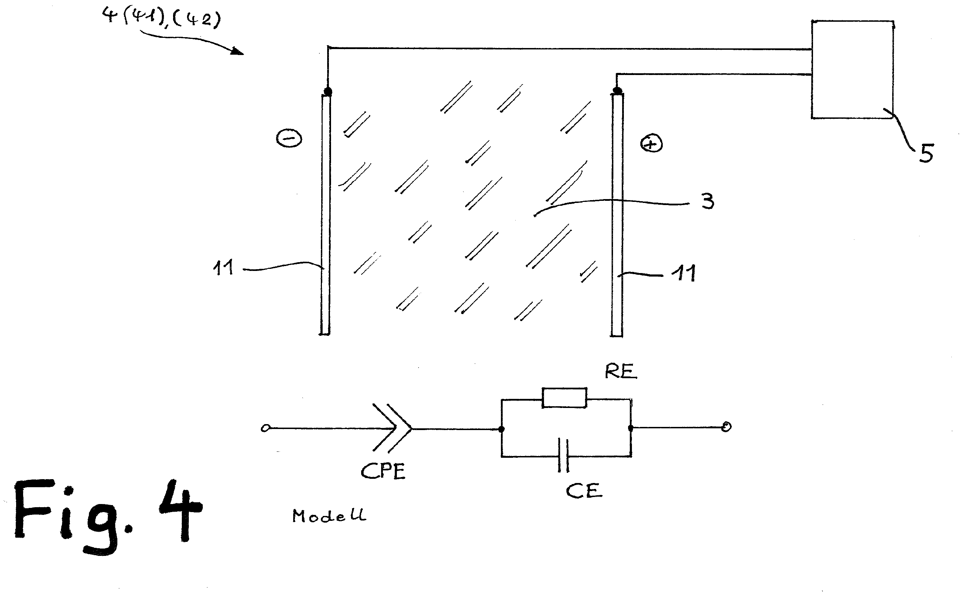

Der grundsätzliche Aufbau und die entsprechende Wirkungsweise wurden vorstehend in Verbindung mit den Figuren 1 und 2 beschrieben. In Verbindung mit den weiteren Figuren 3 und 4 werden Einzelheiten zu dem erfindungsgemäßen Verfahren in Verbindung mit der Impedanzspektroskopie beschrieben.

Wird zumindest einer der beiden Sensoren 41 und 42 der Sensoreinrichtung 4 mittels der Steuerungseinrichtung 5 (Ansteuerungseinrichtung 6) mit entsprechenden elektrischen Signalen zur Durchführung von Messungen angesteuert, ergeben sich für einen vorbestimmten Frequenzbereich von beispielsweise 1 mHz bis 120 MHz jeweilige Impedanzkennlinien des Realteils und des Imaginärteils der komplexen Impedanz Z in der Impedanzebene. Derartige Kennlinien können somit getrennt für den Realteil Re(Z) und Imaginärteil Im(Z) der komplexen Impedanz Z über der Frequenz dargestellt werden. Fig. 3 zeigt in diesem Zusammenhang den Verlauf einer kombinierten Impedanzkennlinie, wobei die Abszisse den Realteil der Impedanz Z und die Ordinate den Imaginärteil der Impedanz Z zeigt. Die komplexe Impedanz Z wird zur Vereinfachung nachstehend als Impedanz Z bezeichnet. Aus dem Verlauf der Impedanzkennlinie K (nachstehend vereinfacht als Kennlinie K bezeichnet) ergeben sich einige Eigenschaften des zu erfassenden fluiden Mediums 3 und hier speziell Eigenschaften der zu überprüfenden wässrigen Lösung. In Verbindung mit der Impedanzspektroskopie werden im allgemeinen zur Erfassung eines Werts der Impedanz Z der Realteil Re(Z) und der Imaginärteil Im(Z) der Impedanz oder des Impedanzvektors (gemäß einer graphischen Darstellung) bestimmt, woraus die benötigten Werte wie die Impedanz, ihr Betrag und der Phasenwinkel φ ermittelt werden können.

Bei der Impedanzspektroskopie wird der Impedanzvektor über einen bestimmten Frequenzbereich f (oder einen entsprechenden Bereich von Winkelgeschwindigkeiten ω) zu diskreten Frequenzwerten aufgenommen. Insbesondere wird in Verbindung mit der Impedanzspektroskopie zwischen Strom und Spannung auch der Phasenunterschied zwischen diesen beiden Größen als Phasenwinkel φ erfasst. Der der Impedanzspektroskopie unterzogene Prüfling ist im Wesentlichen die Sensoreinrichtung 4 (eine Sensorzelle oder Messzelle einschließlich des Mediums 3), beispielsweise der erste Sensor 41, wobei eine kapazitive und eine resistive Messung (Leitwertmessung) ermöglicht wird, und wobei die Sensoreinrichtung 4 im Wesentlichen in Form eines Plattenkondensators mit Kondensatorplatten 11 gemäß der schematischen Darstellung in Fig. 1 aufgebaut ist.

Fig. 4 zeigt in diesem Zusammenhang in schematischer und vereinfachter Weise die Platten 11 des Plattenkondensators der Sensoreinrichtung 4 (des ersten Sensors 41) und dazwischen das Medium 3 (Messzelle), das als eine zur Ionenbildung neigende wässrige Lösung vorgesehen ist. Wird zwischen den Platten 11 des Sensors 41 durch Anlegen einer Spannung ein elektrisches Feld ausgebildet, das ein Gleichfeld bei Gleichspannungsgrößen oder ein Wechselfeld bei Wechselspannungsgröße ist, treten in dem Medium 3 und insbesondere bei den verschiedenen Ionen entsprechende Reaktionen auf, so dass das als Dielektrikum dienende Medium 3 die kapazitiven Eigenschaften des Plattenkondensators der Sensoreinrichtung 4 (des ersten Sensors 41) beeinflusst. Diese Beeinflussung äußert sich in veränderlichen elektrischen Werten hinsichtlich der Kapazität des ersten Sensors 41. Fig. 4 zeigt somit ein vereinfachtes Modell, bzw. ein elektrisches (elektrochemisches) Ersatzschaltbild des ersten Sensors 41 in Verbindung mit dem Einfluss von insbesondere Wechselspannungsgrößen auf die dielektrischen Eigenschaften des Mediums 3 (beispielsweise der wässrigen Lösung).

Die einfache Bauform eines Plattenkondensators als Sensoreinrichtung 4 (erste und zweite Sensoren 41 und 42) dient zur Veranschaulichung der prinzipiellen Anordnung und Wirkungsweise des jeweiligen Sensors, wobei weitere abgewandelte Bauformen möglich und für bestimmte Anwendungen zweckmäßig sind. Zur Ansteuerung der Sensoreinrichtung 4 kann die Ansteuerungseinrichtung 6 der Steuerungseinrichtung 5 einen Prüf- oder Testsignalgenerator aufweisen, dessen Frequenz beliebig einstellbar ist und beispielsweise innerhalb eines Frequenzbereichs von 1 mHz bis 120 MHz und beispielsweise von 40 Hz bis 110 MHz einstellbar ist.

Das ebenfalls in Fig. 4 gezeigte Modell (elektrochemisches Modell des Sensors bzw. der Sensor- oder Messzelle) umfasst einen Elektrolytwiderstand RE und eine Elektrolytkapazität CE, die in ihrer dargestellten Parallelschaltung den Einfluss des zu untersuchenden Mediums 3, und hier den Einfluss einer wässrigen Lösung beschreiben. Die Sensoreinrichtung 4, beispielsweise in Form des ersten Sensors 41, wird dargestellt durch eine Impedanz Zcpe und beschreibt den Sensor selbst. Die Impedanz Zcpe betrifft somit den Einfluss der Platten 11 und speziell der sensoraktiven Oberfläche des ersten Sensors 41. Das Sensorelement mit der Impedanz Zcpe wird nachstehend als Sensorelement CPE bezeichnet. Die Messzelle oder die Sensoreinrichtung 4 oder der erste Sensor 41 umfasst das Sensorelement CPE sowie den Elektrolytwiderstand RE und die Elektrolytkapazität CE als Einfluss des Mediums 3. Der Elektrolytwiderstand RE und die Elektrolytkapazität CE betreffen somit den Elektrolytbereich, während das Sensorelement CPE (mit einer Impedanz Zcpe) den Elektrodenbereich und speziell die Platten 11 des Plattenkondensators bzw. des ersten Sensors 41 betrifft.

Insgesamt stellt das (physikalische) Modell bzw. das (elektrochemische) Ersatzschaltbild gemäß Fig. 4 die Eigenschaften der gesamten Messzelle einschließlich des Sensorelements CPE und des Mediums 3 (wässrige Lösung) dar und ermöglicht somit eine Gesamtbetrachtung der jeweiligen Beeinflussungen der Elektroden und des zu untersuchenden Mediums 3.

Der Elektrolytwiderstand RE beschreibt im Wesentlichen die Leitfähigkeit des Mediums 3 in Form der wässrigen Lösung. Infolge des Übergangs zwischen den Platten 11 und dem Medium 3 entstehen kapazitive Komponenten, so dass der Elektrolytwiderstand RE im Allgemeinen nicht auf einfache Weise mit einer Gleichspannungsmessung bestimmt werden kann. Der Elektrolytwiderstand RE lässt sich elektrochemisch als eine Superposition sämtlicher in der wässrigen Lösung (dem Medium 3) gelösten Ionen deuten, die als mobile Ladungsträger innerhalb des Mediums 3 zu betrachten sind.

Die Elektrolytkapazität CE kann in Abhängigkeit davon, wie die Sensoreinrichtung 4 bzw. der erste Sensor 41, aufgebaut ist, mit den hierfür geltenden Grundgleichungen berechnet werden. Im vorliegenden Fall, bei dem zur vereinfachten Darstellung die Anordnung eines Plattenkondensators mit Platten 11 betrachtet wird (Figuren 1 und 4), kann zur überschlägigen Berechnung beispielsweise die Grundgleichung des Plattenkondensators verwendet werden, sofern der Plattenabstand relativ gering ist. Die Erfindung ist hierauf jedoch nicht beschränkt, vielmehr können weitere Kondensator-Anordnungen verwendet werden, wie beispielsweise flächig ausgebildete Kondensatoren mit ringförmig oder parallel ausgebildeten Elektroden. Ebenso besteht die Möglichkeit der Verwendung von Zylinderkondensatoren oder Kondensatoren mit Interdigital-Struktur.

Das Sensorelement CPE mit der Impedanz Zcpe stellt ein sogenanntes Constant-Phase-Element dar und modelliert den Aufbau und das Verhalten einer sogenannten Doppelschicht im Bereich der Elektroden bzw. der Kondensatorplatten 11 gemäß Fig. 4.

Liegt an einer Elektrodenoberfläche ein bestimmtes Potential an, versuchen Ionen (Überschuss-Ionen), die in Elektrolyten (hier beispielsweise im Medium 3) enthalten sind, sich der Elektrodenoberfläche möglichst weit zu nähern. Da die Ionen jedoch von einer Hydrathülle umgeben sind, gelingt diese Annäherung an die Elektrodenoberfläche (aktive Fläche der Platten 11 gemäß 4) nur bis zu einem bestimmten Abstand, der durch die Hydrathülle vorgegeben ist. Ein Austausch der Ladungen wird durch die Solvatisierung der Ionen verhindert, so dass sich eine sogenannte Helmholtz-Doppelschicht an einer jeweiligen Elektrode (Kondensatorplatte) ausbildet. In elektrischer Hinsicht wirkt die Helmholtz-Doppelschicht wie ein Plattenkondensator in Verbindung mit der jeweiligen Platte 11 (Fig. 4). Eine solche Doppelschicht kann im Allgemeinen eine sehr hohe Kapazität aufweisen. Die vorstehenden Betrachtungen folgen einem vereinfachten Modell einer starren Helmholtz-Doppelschicht. Für weitergehende Betrachtungen kann das spezielle Modell für diesen Sachverhalt verfeinert werden.

Das vorliegende Sensorelement Zcpe berücksichtigt im Wesentlichen die vorstehend angegebenen Sachverhalte und die Impedanz Zcpe wird mathematisch beschrieben mit der nachfolgenden Gleichung 1.

$$\underline{Z}_{CPE}(\omega) = \quad 1 \,/\, Y_o(j\omega)^{\alpha} \qquad\qquad \text{Gleichung 1}$$

Der Exponent $\alpha$ wird als CPE-Exponent $\alpha$ und liegt zwischen 1 und 0. Für $\alpha$ = 1 beschreibt das Sensorelement Zcpe einen idealen Kondensator, für $\alpha$ = 0 einen idealen Widerstand. Die Kapazität $Y_0$ ist im physikalischen Sinn keine Kapazität der Einheit F (Farad, As/V). Die Größe $Y_0$ ist eine Konstante mit der Einheit $s^{\alpha}/\Omega$ und kann als Doppelschicht-kapazität betrachtet werden. Unter weiter vereinfachten Betrachtungen, speziell in Verbindung mit einem niedrigen Frequenzbereich von 0.1 Hz bis etwa 10 kHz kann der Exponent $\alpha$ bestimmt werden gemäß der nachfolgenden Gleichung 2. Hierbei kann der Phasenwinkel $\varphi$ = f($\alpha$) für ein reines Constant-Phase-Element aus dem CPE-Exponenten $\alpha$ bestimmt werden.

$$\alpha \;=\; -\text{arg}\,(\,\underline{Z}(\omega)\,)\,{\cdot}2/\pi \qquad\qquad \text{Gleichung 2}$$

Für den im Wesentlichen die Doppelschichtkapazität repräsentierenden Parameter $Y_0$ gilt ferner die Gleichung 3.

$$Y_0 \;=\; 1/\,(|\,\underline{Z} - RE\,|) \qquad\qquad \text{Gleichung 3}$$

Unter Berücksichtigung der Darstellung der Impedanzkennlinie K gemäß Fig. 3 ist der Elektrolytwiderstand RE bestimmt in einem Punkt oder Bereich der Kennlinie K, bei dem der Imaginärteil Im (Z) annähernd oder gleich 0 ist. Dies ist in Fig. 3 dargestellt. Die Impedanz des Systems ist bei dieser Frequenz rein reell und stellt damit den Elektrolytwiderstand RE dar.

Werden an die Sensoreinrichtung 4 oder den ersten Sensor 41 und somit einerseits an die in Fig. 4 gezeigten Platten 11 in Verbindung mit dem Modell der Messzelle die Signale entsprechend der Impedanzspektroskopie angelegt, tritt bei der Erfassung der entsprechenden Impedanzwerte unter Berücksichtigung einer Darstellung in der komplexen Impedanzebene ein jeweiliger Phasenwinkel $\varphi$ auf, wobei der Phasenwinkel $\varphi$ gemäß Gleichung 4 dargestellt werden kann. Der Phasenwinkel $\varphi$ ist von dem CPE-Exponenten $\alpha$ abhängig.

$$\varphi(\alpha) = -\frac{\pi}{2}\alpha \qquad\qquad \text{Gleichung 4}$$

wobei sich durch Auflösen der Gleichung 4 nach $\alpha$ der CPE-Exponent $\alpha$ gemäß der nachfolgenden Gleichung 5 bestimmt zu

$$\alpha = -\varphi \cdot \frac{2}{\pi} \qquad\qquad \text{Gleichung 5}$$

In Verbindung mit dem derart bestimmten CPE-Exponenten $\alpha$ ergibt sich der Zusammenhang zu Gleichung 2.

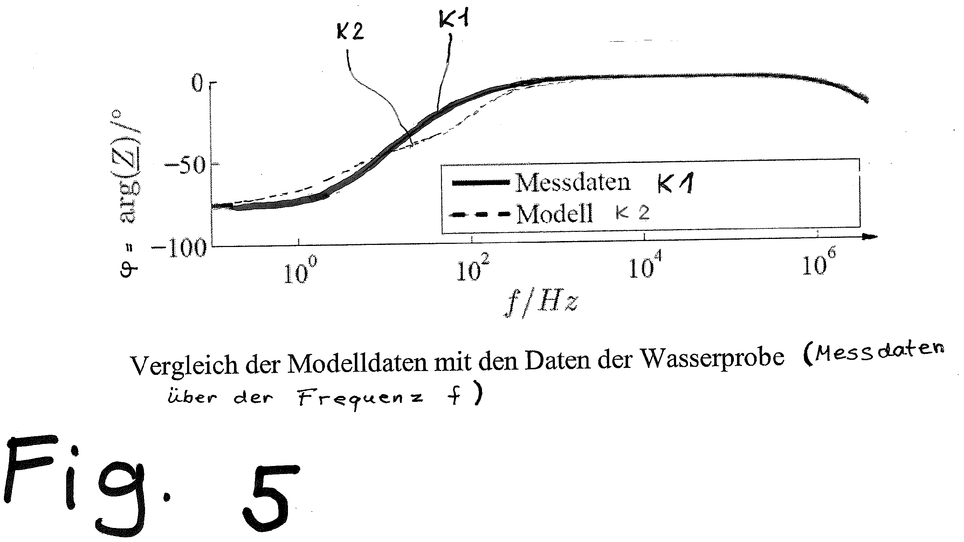

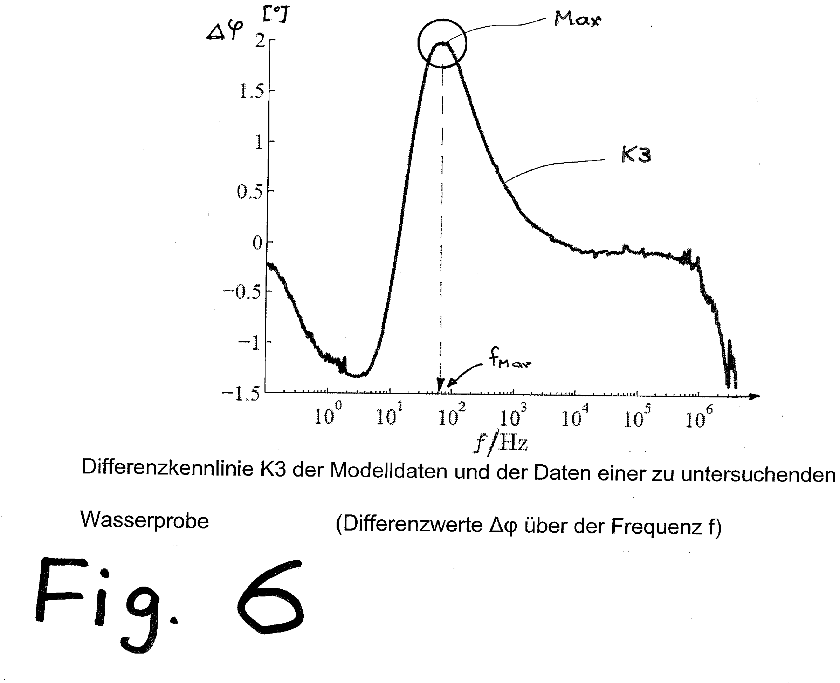

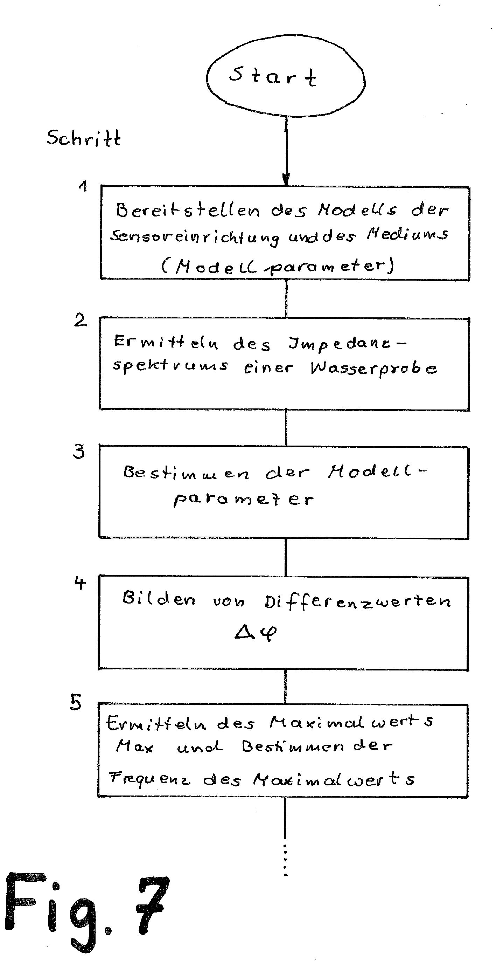

Nachstehend wird zusammenfassend das Verfahren mit einzelnen Schritten zur Erfassung von Eigenschaften wässriger Medien in Verbindung mit den Figuren 5 bis 7 beschrieben. Die Figuren 5 und 6 zeigen nachstehend beschriebene Kennlinien, und Fig. 7 zeigt in der grafischen Darstellung ein Anlaufdiagramm mit jeweiligen Verfahrensschritten. Das Ablaufdiagramm von Fig. 7 zeigt die Schritte in Verbindung mit der Erfassung von Eigenschaften wässriger Medien, wobei sich an die angegebenen Schritte weitere Schritte anschließen können zur ergänzenden Auswertung der Erfassungsergebnisse oder zur Durchführung von zusätzlichen Maßnahmen entsprechend dem Erfassungsergebnis. Auch eine Aufbereitung und Darstellung (Anzeige) des Erfassungsergebnisses sowie eine spezielle Speicherung kann vorgesehen sein.

Entsprechend den vorstehenden Überlegungen wird für eine zu untersuchende Wasserprobe und somit eine bestimmte bekannt Wasserprobe mit einer auf anderen Wegen ermittelten und bestätigten Wasserhärte und insbesondere einer Carbonathärte ein Impedanzspektrum (oder mehrere Messungen) über einen typischen Frequenzbereich von beispielsweise 0.1 Hz bis 4 MHz durchgeführt. Es wird somit die bestimmte Wasserprobe mittels des Impedanzspektrums erfasst und kann in Verbindung mit weiteren Daten ausgewertet werden.

Ferner wird das vorstehend beschriebene physikalische Modell gemäß der Darstellung in Fig. 4 bereitgestellt (erster Schritt) und werden ferner die Modellparameter $Y_0$ zur Berücksichtigung der Doppelschicht-Kapazität (konstanter Parameter) sowie der CPE-Exponent $\alpha$ in Verbindung mit dem Modell und dem Impedanzspektrum der bekannten Wasserprobe ermittelt (zweiter und dritter Schritt). Diese Parameter stellen Werte dar, die individuell und frequenzabhängig für die jeweilige (die bestimmte) Wasserprobe bestehen und im Modell (elektrochemisches Ersatzschaltbild) gemäß Fig. 4 verwendet werden. Die vorstehend angegebene bestimmte Wasserprobe umfasst zumindest eine Wasserprobe mit bekannten Eigenschaften (z. B. Wasserhärte). Vorzugsweise werden mehrere bekannte Wasserproben zur Parameterbestimmung herangezogen, und weiter vorzugsweise eine Vielzahl von bekannten Wasserproben, wobei einzelnen Werte, die aus den jeweiligen Wasserproben hervorgehen, gemittelt werden, um beispielsweise den Einfluss von Ungenauigkeiten bei den Erfassungen zu vermindern.

Im Ergebnis ist grundsätzlich eine Verbindung zwischen der bestimmten bekannten Wasserprobe (oder den mehreren oder vielen bekannten Wasserproben) und dem Modell hergestellt, und es sind die relevanten Modellparameter, wie die Größe $Y_0$ und der CPE-Exponent $\alpha$ bestimmt, so dass eine direkte Korrelation zwischen dem Modell mit seinen Parametern und der Wasserprobe in Verbindung mit der Messung besteht.

In einem weiteren (vierten) Schritt erfolgt zumindest eine Messung einer weiteren Wasserprobe (oder auch mehrere Messungen für dieselbe oder verschiedene Wasserproben), wobei in Verbindung mit der Anwendung der Impedanzspektroskopie (siehe Fig. 3) Realdaten gebildet werden und zur Auswertung zur Verfügung stehen. Diese Wasserproben gemäß dem vierten Schritt stellen unbekannte zu erfassenden Wasserproben dar (aktuelle Wasserproben).

Mit den zur Verfügung stehenden Realdaten zumindest einer weiteren aktuellen Wasserprobe (aktuelle Probenparameter) kann eine jeweilige Kennlinie dargestellt werden, wie sie beispielsweise in Fig. 5 gezeigt ist. Gemäß Fig. 5 mit einer Darstellung des Phasenwinkels $\varphi$ über der Frequenz f ist ein Vergleich von Modelldaten (aus einer Simulation des Modell mittels den Frequenzen oder Frequenzbereichen entsprechend der Impedanzspektroskopie), die zuvor an der bestimmten Wasserprobe verifiziert wurden, und Daten einer aktuellen Wasserprobe (aus der Impedanzspektroskopie) dargestellt. Die durchgezogene Linie der Messdaten der aktuellen Wasserprobe (im vorliegenden Fall beispielsweise mit einer vorbestimmten Wasserhärte) in Form der Kennlinie K1 zeigt Bereiche geringer Abweichung zu der gestrichelten Kennlinie K2, die aus dem Modell (Fig. 4) und den entsprechenden Modellparametern hervorgeht. Es liegt dieser Darstellung der vorstehend angegebene Frequenzbereich zugrunde. Es werden somit in dem angegeben vierten Schritt die Impedanzspektroskopie-Daten der weiteren Messung sowie Differenzen bzw. Differenzwerte $\Delta\varphi$ dieser erfassten Daten zu den aus dem Modell hervorgehenden Daten gebildet.

Des Weiteren werden über den genannten und in Fig. 5 dargestellten Frequenzbereich die jeweiligen Abweichungen zwischen den Daten des Modells (Ersatzschaltbild, Fig. 4, Simulationsdaten) und derjenigen der realen Messung einer bestimmten unbekannten Wasserprobe auf der Basis der Impedanzspektroskopie ermittelt und in Form einer dritten Kennlinie K3 dargestellt. Ein Beispiel für die dritte Kennlinie K3 ist in Fig. 6 gezeigt und stellt somit eine Differenzkennlinie der Modelldaten und der Daten der aktuellen (d. h. unbekannten) Wasserprobe dar. Die aktuelle Wasserprobe bildet somit eine zu untersuchende Wasserprobe, d.h. eine unbekannte Wasserprobe, deren Gesamthärte oder zumindest deren Carbonathärte zu bestimmen ist. Fig. 5 zeigt prinzipiell den Phasenwinkel $\varphi = f(\alpha)$ (Gleichungen 4 und 5) jeweils für die Modelldaten und entsprechenden Daten der Wasserprobe.

Fig. 6 zeigt demgegenüber die dritte Kennlinie K3 als Differenzkennlinie der gemessenen Werte $(\arg(Z_{Wasserprobe}))$ über dem Frequenzbereich der Impedanzspektroskopie, von denen bezüglich der jeweiligen Frequenzen die entsprechenden Modelldaten $(\arg(Z_{Modell}))$ subtrahiert werden, so dass sich die Differenzkennlinie K3 aus Differenzwerten $\Delta\varphi$ gemäß Gleichung 6 ergibt.

$$\Delta\varphi = \arg(Z_{Residuen}) = \arg(Z_{Wasserprobe}) - \arg(Z_{Modell}) \qquad \text{Gleichung 6}$$

In der Darstellung in Fig. 6 zeigt die Differenzkennlinie oder dritte Kennlinie K3 einen Maximalwert Max der Differenzwerte $\Delta\varphi$ bei einer bestimmten Frequenz oder bei einem kleinen Frequenzbereich. Die Genauigkeit der Bestimmung des Maximalwerts Max hängt im Wesentlichen davon ab, mit welchen mathematischen Algorithmen der Maximalwert Max (bzw. sein Bereich) bestimmt werden kann. Der Maximalwert Max wird in einem weiteren (fünften) Schritt bestimmt.

Die Abweichungen oder Differenzen bzw. Differenzwerte $A\varphi$, wie sie in Verbindung mit Fig. 6 durch die Differenzkennlinie oder dritte Kennlinie K3 dargestellt sind, werden auch als Modellresiduen bezeichnet. Die vorstehend beschriebene Analyse der Modellresiduen stellt Angaben über den Hydrogencarbonatgehalt (Hydrogencarbonat-Konzentration $c(HCO_3^-)$) einer entsprechenden unbekannten Wasserprobe (gemäß den Figuren 5 und 6 der aktuellen Wasserprobe) dar. Aus der dritten Kennlinie K3 gemäß Fig. 6 geht hervor, dass im unteren Frequenzbereich zunächst ein Tiefpunkt und danach ein Hochpunkt mit dem Maximum bzw. Maximalwert Max auftritt. Fig. 6 zeigt somit einen charakteristischen Verlauf der Residuen des Arguments der Impedanz Z (komplexe Impedanz). Bei der Betrachtung des Maximums Max mehrerer Wasserproben ergibt sich, dass die Frequenz, bei der das Maximum Max der Differenzkennlinie oder dritten Kennlinie K3 auftritt (Phasen-Residuen-Maximum) mit der Hydrogencarbonationen-Konzentration $c(HCO_3^-)$ korreliert. Die Frequenz f (in Hz) korreliert somit direkt mit der Konzentration der Hydrogencarbonationen $HCO_3^-$ und stellt somit ein Maß für die Hydrogencarbonationen-Konzentration $c(HCO3^-)$ dar.

Gemäß Fig. 6 ist erkennbar, dass der Bereich des Maximalwerts oder Maximums Max der dritten Kennlinie K3 (Differenzkennlinie, Differenzwerte $\Delta\varphi$) einen bestimmten Bereich einnimmt. Die Genauigkeit des Erfassungsergebnisses und somit die Genauigkeit der Bestimmung der Frequenz des Maximums der Residuen gemäß Fig. 6 hängt von der Genauigkeit der Bestimmung des Maximums Max in Verbindung mit vorbestimmten bzw. geeigneten mathematischen Algorithmen ab.

Mittels der vorstehend beschriebenen impedanzspektroskopischen Messungen und in Verbindung mit einem entsprechenden physikalischen oder elektrochemischen Modell gemäß der Darstellung in Fig. 4 können die Gesamthärte und die Hydrogencarbonationen-Konzentration $c(HCO3^-)$ einer unbekannten bzw. zu erfassenden Wasserprobe mit den angegebenen Schritten des Verfahrens (gemäß Fig. 7) bestimmt werden. Speziell bezieht sich die vorliegende Erfindung auf die Auswertung von Modellresiduen, d.h. auf die Bestimmung der Differenzkennlinie K3 zwischen realen erfassten Daten einer Wasserprobe eines zu untersuchenden Wassers und den zuvor verifizierten Daten des physikalischen Modells (mittels Impedanzspektroskopie simuliertes Modell), wie es in Fig. 4 gezeigt ist. Es kann somit in Verbindung mit der Auswertung der Differenzkennlinie der Modellresiduen (Figuren 5 und 6) die Hydrogencarbonationen-Konzentration $c(HCO3^-)$ bestimmt werden, da diese zu einem charakteristischen und damit auswertbaren Verlauf der Modellresiduen gemäß Fig. 6 führt. Es besteht des Weiteren ein Zusammenhang zwischen der Gesamthärte einer Wasserprobe und der Konzentration der Hydrogencarbonationen $c(HCO3^-)$, so dass auf der Basis der Kenntnis der Konzentration der Hydrogencarbonationen $c(HCO3^-)$ auch auf die Gesamtwasserhärte (mit vorbestimmter Genauigkeit) geschlossen werden kann. Ebenso besteht ein Zusammenhang zwischen der Gesamthärte und einem Elektrolytleitwert.

Die vorstehend beschriebenen Schritte gemäß Fig. 7 werden grundsätzlich von der Steuerungseinrichtung 5 veranlasst und durchgeführt, indem Signale an die Sensoreinrichtung 4 (den ersten Sensor 41 und/oder den zweiten Sensor 42) oder die Messzelle angelegt und Antwortsignale aufgenommen und ausgewertet werden. Somit umfasst die Steuerungseinrichtung die Ansteuerungseinrichtung 6 zur Bereitstellung der Signale zur Durchführung einer Messung mittels Impedanzspektroskopie. Die Steuerungseinrichtung 5 in Verbindung mit der Ansteuerungseinrichtung 6 bildet somit eine Einrichtung zur Erfassung des Impedanzspektrums einer vorbestimmten zu untersuchenden Wasserprobe. Hierbei werden die Antwortsignale aufgenommen und ausgewertet.

Auf der Basis des in Fig. 4 gezeigten Modells, das in der Steuerungseinrichtung 5 hinterlegt ist, werden die Modellparameter, im vorliegenden Fall $Y_0$ und $\alpha$ mit einer jeweiligen Programmsteuerung anhand des zuvor bestimmten Impedanzspektrums und des hinterlegten Modells ermittelt und hinzugefügt, so dass die Steuerungseinrichtung 5 sämtliche zur Durchführung dieser Maßnahmen erforderlichen Einrichtungen aufweist.

Die Steuerungseinrichtung 5 umfasst ferner eine Einrichtung zur Bildung der jeweiligen Kennlinien (erste und zweite Kennlinie K1 und K2), wie sie in Fig. 5 gezeigt sind. Die Kennlinien können hierbei mittels entsprechender Einrichtungen gebildet werden, wobei hinsichtlich der Verarbeitung und Speicherung der Daten Kennfelder und Datentabellen vorgesehen sein können. Die Steuerungseinrichtung 5 umfasst ebenfalls eine Differenzbildungseinrichtung zur Bildung einer Differenz zwischen den realen Daten einer Wasserprobe (beispielsweise einer aktuellen Wasserprobe eines zu untersuchenden Wassers) und den Daten entsprechend dem hinterlegten Modell (Modell oder Ersatzschaltbild gemäß Fig. 4), und somit Differenzen, wie sie auch aus einem Vergleich der ersten und zweiten Kennlinien K1 und K2 hervorgehen. Ebenso besteht eine Einrichtung zur Erzeugung und Speicherung der Differenzdaten, mittels denen die Differenzkennlinie oder dritte Kennlinie K3 gemäß Fig. 6 gebildet wird.

Auf der Basis dieser Differenzwerte und entsprechend der Darstellung der Differenzwerte in der Differenzkennlinie K3 besteht eine Einrichtung zur Bestimmung eines Maximums dieser Daten oder der Differenzkennlinie K3, wobei zu dem Maximum oder Maximalwert Max eine jeweilige Frequenz $f_{Max}$ (fallweise auch ein kleiner Frequenzbereich) bestimmt wird. Falls ein Frequenzbereich bestimmt wird, besteht die Möglichkeit, auf der Basis des Frequenzbereichs einen Mittelwert als die Frequenz $f_{Max}$ des Maximums oder Maximalwerts Max zu bestimmen, so dass zur Bestimmung der Hydrogencarbonationen-Konzentration $c(HCO3^-)$ ein eindeutiger Frequenzwert vorliegt.

Die Steuerungseinrichtung 5 weist somit sämtliche Einrichtungen zur Durchführung des vorstehend beschriebenen Verfahrens auf.

Das vorstehend beschriebene Verfahren und die zugehörige Vorrichtung erlauben, die Bestimmung der Hydrogencarbonationen-Konzentration $c(HCO3^-)$ auf relativ einfache Weise und mit einer ausreichenden Genauigkeit, wobei Rückschlüsse auf die Gesamtionenkonzentration (Gesamtwasserhärte) möglich sind. Die Erfassung der Eigenschaften einer Wasserprobe und insbesondere der Hydrogencarbonationen-Konzentration steht im Zusammenhang mit einem vereinfachten elektrochemischen Ersatzschaltbild oder Modell (Fig. 4), das das elektrische Verhalten der Sensoreinrichtung und der elektrochemischen Eigenschaften der zu überprüfenden Wasserprobe berücksichtigt. In Verbindung mit einer Optimierung bzw. Anpassung des Modells werden die Parameter des Modells (Modellparameter $Y_0$ zur Berücksichtigung der Doppelschicht-Kapazität sowie der CPE-Exponent a) mit Erfassungs- oder Messdaten einer bekannten Wasserprobe bestimmt, so dass auf der Basis des verfeinerten Modells weitere Wasserproben mit unbekannten Eigenschaften bewertet werden können.

Die erfassten Werte der Hydrogencarbonationen-Konzentration c(HCO3$^-$) können die Grundlage für vielfältige Steuerungs- und Regelungsvorgänge darstellen, wie beispielsweise als Grundlageninformation für kommunale Wasserwerke, oder bei sämtlichen Anwendungen von Frischwasser mit einer bestimmten Wasserhärte, wenn diese für nachfolgende Prozesse wichtig oder auch nachteilig sein kann. In Kenntnis der Hydrogencarbonationen-Konzentration können speziell Waschvorgänge hinsichtlich der Waschmittelkonzentration und weiteren Parametern eines Waschvorgangs günstig beeinflusst werden, so dass die Möglichkeit besteht, beispielsweise die Menge des eingesetzten Waschmittels und einen Energieeinsatz zu vermindern. Ebenso können Kenntnisse über die Wasserhärte ein Maß darstellen, wann Wartungs- oder Reinigungsarbeiten für bestimmte Einrichtungen erforderlich sind. In jedem Fall kann die vorstehend beschriebene Vorrichtung mit dem zugehörigen Verfahren in handelsüblichen Wascheinrichtungen eingebaut oder auch nachgerüstet werden, so dass die Wascheinrichtung ohne Zutun einer Bedienperson eine Erfassung der Wassereigenschaften des Frischwassers auf einfache und kostengünstige Weise durchführen kann.

## Patentansprüche

1. Verfahren zur Erfassung von Eigenschaften eines wässrigen Mediums (3) mittels einer Sensoreinrichtung (4, 41) und in Verbindung mit einer Impedanzspektroskopie, mit den Schritten:

   - Bereitstellen eines Modells der Sensoreinrichtung (4, 41) und des zu erfassenden Mediums (3) mit den jeweiligen Modellparametern ($Y_0$, $\alpha$) (Schritt S1),
   - Ermitteln eines Impedanzspektrums einer vorbestimmten Probe des Mediums (3) mit bekannten Eigenschaften über einen vorbestimmten Frequenzbereich und Bereitstellen von Daten in Form der Impedanz oder des Impedanzvektors (Schritt S2),
   - Bestimmen der Modellparameter ($Y_0$, $\alpha$) auf der Basis der Daten des Impedanzspektrums der vorbestimmten Probe und Bilden von Modelldaten aus einer Simulation des Modells mit den Modellparametern über dem vorbestimmten Frequenzbereich (Schritt S3),
   - Bilden von Differenzwerten zwischen den Daten aus dem Modell mit den Modellparametern ($Y_0$, $\alpha$) und erfassten Daten des Impedanzspektrums einer weiteren Probe des Mediums (3) über dem vorbestimmten Frequenzbereich (Schritt S4), und
   - Ermitteln eines Maximalwerts (Max) der Differenzwerte ($\Delta\varphi$) und Bestimmen der Frequenz (fMax) des Maximalwerts (Max) aus dem vorbestimmten Frequenzbereich als Maß für die Eigenschaften des Mediums (3) (Schritt S5).

2. Verfahren nach Anspruch 1, wobei das Modell ein Constant-Phase-Element (CPE) aufweist zur modellmäßigen Darstellung der Eigenschaften der Sensoreinrichtung (4,41).

3. Verfahren nach Anspruch 2, wobei die Modellparameter des Modells einen ersten Parameter ($Y_0$) zur Berücksichtigung einer Doppelschicht-Kapazität der Sensoreinrichtung (4, 41) und einen zweiten Parameter ($\alpha$) zur Berücksichtigung eines Phasenwinkels ($\varphi$) des Impedanzspektrums in dem vorbestimmten Frequenzbereich aufweist.

4. Verfahren nach einem der Ansprüche 1 bis 3, wobei das Ermitteln des Impedanzspektrums das Ermitteln von Impedanzwerten der Sensoreinrichtung (4, 41) unter dem Einfluss des Mediums (3) im vorbestimmten Frequenzbereich umfasst.

5. Verfahren nach einem der Ansprüche 1 bis 3, wobei der Schritt (S4) des Bildens von Differenzwerten das Erfassen von Impedanzwerten eines Impedanzspektrums einer unbekannten Probe des Mediums (3) in dem vorbestimmten Frequenzbereich umfasst.

6. Verfahren nach Anspruch 5, wobei der Schritt (S4) des Bildens von Differenzwerten den Schritt des Bildens der Differenzwerte aus den simulierten Daten des Modells und den erfassten Impedanzwerten der unbekannten Probe des Mediums (3) für eine jeweilige Frequenz aus dem vorbestimmten Frequenzbereich umfasst.

7. Verfahren nach Anspruch 1 oder 2, wobei das Modell eine Parallelschaltung eines Widerstandselements (RE) zur Berücksichtigung des Elektrolytwiderstands, sowie ein kapazitives Element (CE) zur Berücksichtigung der Elektrolytkapazität des Mediums (3) aufweist.

8. Verfahren nach einem der Ansprüche 1 bis 7, wobei zur Überprüfung mehrere Proben des Mediums (3) jeweils eine Wiederholung der Schritte S1 bis S5 erfolgt.

9. Vorrichtung zur Durchführung des Verfahrens zur Erfassung von Eigenschaften eines wässrigen Mediums (3) mittels einer Sensoreinrichtung (4, 41) und in Verbindung mit einer Impedanzspektroskopie, mit:

- einer Einrichtung (5) zum Bereitstellen eines Modells der Sensoreinrichtung (4, 41) und des zu erfassenden Mediums (3) mit den jeweiligen Modellparametern ($Y_0$, $\alpha$) (Schritt S1),
- einer Einrichtung (5) zum Ermitteln eines Impedanzspektrums einer vorbestimmten Probe des Mediums (3) mit bekannten Eigenschaften über einen vorbestimmten Frequenzbereich und Bereitstellen von Daten in Form der Impedanz oder des Impedanzvektors (Schritt S2),
- einer Einrichtung (5) zum Bestimmen der Modellparameter ($Y_0$, $\alpha$) auf der Basis der Daten des Impedanzspektrums der vorbestimmten Probe und Bilden von Modelldaten aus einer Simulation des Modells mit den Modellparametern über dem vorbestimmten Frequenzbereich (Schritt S3),
- einer Einrichtung (5) zum Bilden von Differenzwerten zwischen den Daten aus dem Modell mit den Modellparametern ($Y_0$, $\alpha$) und erfassten Daten des Impedanzspektrums einer weiteren Probe des Mediums (3) über dem vorbestimmten Frequenzbereich (Schritt S4), und
- einer Einrichtung (5) zum Ermitteln eines Maximalwerts (Max) der Differenzwerte ($\Delta\varphi$) und Bestimmen der Frequenz (fMax) des Maximalwerts (Max) aus dem vorbestimmten Frequenzbereich als Maß für die Eigenschaften des Mediums (3) (Schritt S5).

**Claims**

1. Method for detecting properties of an aqueous medium (3) by means of a sensor device (4, 41) and in conjunction with an impedance spectroscopy, comprising the steps:

- providing a model of the sensor device (4, 41) and of the medium (3) to be detected with the respective model parameters (Yo, $\alpha$) (step S1),
- determining an impedance spectrum of a predetermined sample of the medium (3) having known properties over a predetermined frequency range and providing data in the form of the impedance or the impedance vector (step S2),
- determining the model parameters (Yo, $\alpha$) on the basis of the data of the impedance spectrum of the predetermined sample and forming model data from a simulation of the model with the model parameters above the predetermined frequency range (step S3),
- forming difference values between the data from the model with the model parameters (Yo, $\alpha$) and acquired data of the impedance spectrum of a further sample of the medium (3) above the predetermined frequency range (step S4), and
- determining a maximum value (Max) of the difference values ($\Delta\varphi$) and determining the frequency (fMax) of the maximum value (Max) from the predetermined frequency range as a measure of the properties of the medium (3) (step S5).

2. Method according to claim 1, wherein the model comprises a constant phase element (CPE) for model representation of the characteristics of the sensor device (4,41).

3. Method according to claim 2, wherein the model parameters of the model have a first parameter ($Y_0$) for taking into account a double layer capacitance of the sensor device (4, 41) and a second parameter ($\alpha$) for taking into account a phase angle ($\varphi$) of the impedance spectrum in the predetermined frequency range.

4. Method according to one of claims 1 to 3, wherein determining the impedance spectrum comprises determining impedance values of the sensor device (4, 41) under the influence of the medium (3) in the predetermined frequency range.

5. Method according to one of claims 1 to 3, wherein the step (S4) of forming difference values comprises detecting impedance values of an impedance spectrum of an unknown sample of the medium (3) in the predetermined frequency range.

6. Method according to claim 5, wherein the step (S4) of forming difference values comprises the step of forming the difference values from the simulated data of the model and the detected impedance values of the unknown sample of the medium (3) for a respective frequency from the predetermined frequency range.

7. Method according to claim 1 or 2, wherein the model comprises a parallel connection of a resistance element (RE) for taking into account the electrolyte resistance, and a capacitive element (CE) for taking into account the electrolyte capacity of the medium (3).

8. Method according to one of claims 1 to 7, wherein for testing several samples of the medium (3), steps S1 to S5 are repeated in each case.

9. Device for carrying out the method for detecting properties of an aqueous medium (3) by means of a sensor device (4, 41) and in conjunction with an impedance spectroscopy, with:

- means (5) for providing a model of the sensor device (4, 41) and of the medium (3) to be detected with the respective model parameters ($Y_0$, $\alpha$) (step S1),
- means (5) for determining an impedance spectrum of a predetermined sample of the medium (3) having known properties over a predetermined frequency range and providing data in the form of the impedance or the impedance vector (step S2),
- means (5) for determining the model parameters ($Y_0$, $\alpha$) on the basis of the data of the impedance spectrum of the predetermined sample and forming model data from a simulation of the model with the model parameters above the predetermined frequency range (step S3),
- means (5) for forming difference values between the data from the model having the model parameters ($Y_0$, $\alpha$) and acquired data of the impedance spectrum of a further sample of the medium (3) above the predetermined frequency range (step S4), and
- means (5) for determining a maximum value (Max) of the difference values ($\Delta\varphi$) and determining the frequency (fMax) of the maximum value (Max) from the predetermined frequency range as a measure of the properties of the medium (3) (step S5).

**Revendications**

1. Procédé d'enregistrement de propriétés d'un fluide (3) aqueux au moyen d'un dispositif de capteur (4, 41) et en liaison avec une spectroscopie par impédance, avec les étapes de :

- la fourniture d'un modèle du dispositif de capteur (4, 41) et du fluide (3) à enregistrer avec les paramètres de modèle ($Y_0$, $\alpha$) respectifs (étape S1),
- la définition d'un spectre d'impédance d'un échantillon prédéterminé du fluide (3) avec des propriétés connues sur une plage de fréquence prédéterminée et la fourniture de données sous la forme de l'impédance ou du vecteur d'impédance (étape S2),
- la détermination des paramètres de modèle ($Y_0$, $\alpha$) sur la base des données du spectre d'impédance de l'échantillon prédéterminé et la formation de données de modèle à partir d'une simulation du modèle avec les paramètres de modèle sur la plage de fréquence prédéterminée (étape S3),
- la formation de valeurs de différence entre les données à partir du modèle avec les paramètres de modèle ($Y_0$, $\alpha$) et les données enregistrées du spectre d'impédance d'un autre échantillon du fluide (3) sur la plage de fréquence prédéterminée (étape S4), et
- la définition d'une valeur maximale (Max) des valeurs de différence ($\Delta\varphi$) et la détermination de la fréquence (fMax) de la valeur maximale (Max) à partir de la plage de fréquence prédéterminée en tant que mesure pour les propriétés du fluide (3) (étape S5).

2. Procédé selon la revendication 1, dans lequel le modèle présente un élément à phase constante (CPE) pour la représentation selon le modèle des propriétés du dispositif de capteur (4, 41).

3. Procédé selon la revendication 2, dans lequel les paramètres de modèle du modèle présentent un premier paramètre ($Y_0$) destiné à tenir compte d'une capacité à double couche du dispositif de capteur (4, 41) et un second paramètre ($\alpha$) destiné à tenir compte d'un angle de phase ($\varphi$) du spectre d'impédance dans la plage de fréquence prédéterminée.

4. Procédé selon l'une quelconque des revendications 1 à 3, dans lequel la définition du spectre d'impédance comprend la définition de valeurs d'impédance du dispositif de capteur (4, 41) sous l'influence du fluide (3) dans la plage de fréquence prédéterminée.

5. Procédé selon l'une quelconque des revendications 1 à 3, dans lequel l'étape (S4) de la formation de valeurs de différence comprend l'enregistrement de valeurs d'impédance d'un spectre d'impédance d'un échantillon inconnu du fluide (3) dans la plage de fréquence prédéterminée.

6. Procédé selon la revendication 5, dans lequel l'étape (S4) de la formation de valeurs de différence comprend l'étape de la formation des valeurs de différence à partir des données simulées du modèle et des valeurs d'impédance enregistrées de l'échantillon inconnu du fluide (3) pour une fréquence respective de la plage de fréquence prédéterminée.

7. Procédé selon la revendication 1 ou 2, dans lequel le modèle présente un circuit parallèle d'un élément de résistance (RE) destiné à tenir compte de la résistance électrolytique, ainsi qu'un élément capacitif (CE) destiné à tenir compte de la capacité électrolytique du fluide (3).

8. Procédé selon l'une quelconque des revendications 1 à 7, dans lequel pour la vérification de plusieurs échantillons du fluide (3) une répétition des étapes S1 à S5 est respectivement effectuée.

9. Dispositif de réalisation du procédé pour l'enregistrement de propriétés d'un fluide (3) aqueux au moyen d'un dispositif de capteur (4, 41) et en liaison avec une spectroscopie par impédance, avec :

- un dispositif (5) pour la fourniture d'un modèle du dispositif de capteur (4, 41) et du fluide (3) à enregistrer avec les paramètres de modèle ($Y_0$, $\alpha$) respectifs (étape S1),
- un dispositif (5) pour la définition d'un spectre d'impédance d'un échantillon prédéterminé du fluide (3) avec des propriétés connues sur une plage de fréquence prédéterminée et la fourniture de données sous la forme de l'impédance ou du vecteur d'impédance (étape S2),
- un dispositif (5) pour la détermination des paramètres de modèle ($Y_0$, $\alpha$) sur la base des données du spectre d'impédance de l'échantillon prédéterminé et la formation de données de modèle à partir d'une simulation du modèle avec les paramètres de modèle sur la plage de fréquence prédéterminée (étape S3),
- un dispositif (5) pour la formation de valeurs de différence entre les données à partir du modèle avec les paramètres de modèle ($Y_0$, $\alpha$) et les données enregistrées du spectre d'impédance d'un autre échantillon du fluide (3) sur la plage de fréquence prédéterminée (étape S4), et
- un dispositif (5) pour la définition d'une valeur maximale (Max) des valeurs de différence ($\Delta\varphi$) et la détermination de la fréquence (fMax) de la valeur maximale (Max) à partir de la plage de fréquence prédéterminée en tant que mesure pour les propriétés du fluide (3) (étape S5).

Fig. 1

EP 2 902 774 B1

Fig. 2

Fig. 3

EP 2 902 774 B1

4 (41), (42)

−

3

11

11

+

5

RE

CPE

CE

Modell

# Fig. 4

Vergleich der Modelldaten mit den Daten der Wasserprobe (Messdaten über der Frequenz f )

# Fig. 5

EP 2 902 774 B1

Differenzkennlinie K3 der Modelldaten und der Daten einer zu untersuchenden

Wasserprobe                    (Differenzwerte Δφ über der Frequenz f)

# Fig. 6

EP 2 902 774 B1

Start

Schritt

1. Bereitstellen des Modells der Sensoreinrichtung und des Mediums (Modellparameter)

2. Ermitteln des Impedanz-spektrums einer Wasserprobe

3. Bestimmen der Modell-parameter

4. Bilden von Differenzwerten $\Delta\varphi$

5. Ermitteln des Maximalwerts Max und Bestimmen der Frequenz des Maximalwerts

# Fig. 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DD 217557 A1
- DE 19755418 A1
- DE 4111064 A1
- DE 60307108 T2
- US 20050179449 A1

**In der Beschreibung aufgeführte Nicht-Patentliteratur**

- **U. TRÖLTZSCH.** Anwendungspotential der Impedanzspekroskopie für die Waschlaugensensorik. *16. GMA/ITG-Fachtagung Sensoren und Messsysteme,* 2012, 650-661